# EUROPÄISCHE PATENTANMELDUNG

(11) **EP 1 297 791 A1**
(43) Veröffentlichungstag der Anmeldung: **02.04.2003**
(21) Anmeldenummer: 01121570.4
(22) Anmeldetag: 10.09.2001
(51) Int. Cl.: A61B 17/32, A61B 17/16

(54) **Chirurgische Gewebestanze und diese enthaltender Instrumentensatz**

(71) Anmelder: Waldemar Link (GmbH & Co.), 22339 Hamburg (DE)
(72) Erfinder: Link, Helmut D., 22397 Hamburg (DE)
(74) Vertreter: Glawe, Delfs, Moll & Partner

(57) **Zusammenfassung**

Chirurgische Gewebestanze mit einem eine Absaugöffnung (9) aufweisenden Gewebeaufnahmekanal (8). Um die Entfernung von im Gewebeaufnahmekanal (8) angesammeltem Gewebe zu erleichtern, ist die Absaugöffnung (9) mit einer Saugdüsenansatzfläche (15, 16) versehen. Diese kann beispielsweise konisch ausgebildet oder von einem zylindrischen Stutzen gebildet sein. Eine Kupplungseinrichtung zum Ankuppeln eines Absauggeräts kann vorgesehen sein. Die Erfindung bezieht sich ferner auf einen Instrumentensatz, der eine derartige Gewebestanze sowie ein Absauggerät umfaßt.

## Beschreibung

Zum dosierten und lokalisierten Entfernen von Gewebe, insbesondere Knorpel- und Knochengewebe, verwendet man in der Chirurgie sogenannte Gewebe- oder Knochenstanzen. Sie bestehen aus relativ zueinander bewegten Schneidelementen. Sie enthalten einen Raum zur Aufnahme des entfernten Gewebes. Dieser ist als Kanal ausgebildet, der an seinem den Schneidelementen fernen Ende eine Öffnung für den Austritt des entfernten Gewebes aufweist, die im folgenden als Absaugöffnung bezeichnet wird. Bei bekannten Instrumenten dieser Art (DE-U-94 01 494, DE-A-41 15 937, WO 99/55242) führt der Kanal oder ein Kanalabzweig schräg zu dieser Öffnung hin, die eine langgestreckte Form aufweist. Wenn der Kanal voll ist, entleert man ihn durch Öffnen des Instruments und/oder dadurch, daß man die Saugdüse eines Absauggeräts in die Nähe der Absaugöffnung bringt. Je nach Geschick gelingt dies mehr oder weniger leicht. Der Erfindung liegt die Aufgabe zugrunde, die Entleerung des Kanals durch Absaugung zu erleichtern.

Die erfindungsgemäße Lösung besteht in den Merkmalen des Anspruchs 1 und vorzugsweise denjenigen der Unteransprüche. Demgemäß ist vorgesehen, daß die Absaugöffnung mit einer Saugdüsenansatzfläche versehen ist. Das ist eine Anschlagoder Führungsfläche, an der sich die Saugdüse eines Absauggeräts bequem so ansetzen läßt, daß sie die richtige Position in bezug auf die Absaugöffnung hat und ihre Saugwirkung sich voll entfalten kann. Ein dichter Anschluß ist dafür nicht unbedingt erforderlich, wenngleich vorteilhaft.

Die Saugdüsenansatzfläche kann, um die Funktion der Lokalisierung der Saugdüse erfüllen zu können, jegliche Gestalt haben, die zur Positionierung der Saugdüse in funktioneller Nähe der Absaugöffnung geeignet ist. Besonders vorteilhaft ist die Ausrüstung der Absaugöffnung mit einem vorragenden Stutzen. Je nach Größe dieses Stutzens bzw. der Saugdüse kann letztere durch die Bohrung des Stutzens oder dessen Außenfläche geführt werden. Wenn die Durchmesser des Stutzens bzw. der Saugdüse aufeinander abgestimmt sind, kann dadurch auch ein passender, im wesentlichen dichter Anschluß erzielt werden.

Bei einer anderen vorteilhaften Ausführungsform der Erfindung ist die Saugdüsenansatzfläche konisch ausgebildet. Wenn sie hohlkonisch ist, kann die Saugdüse innerhalb des Hohlkonus lokalisiert werden. Wenn die Saugdüse achsgleich mit dem Hohlkonus an diesen angesetzt wird, ergibt sich auch ein im wesentlichen dichter Anschluß, ohne daß es auf die Einhaltung eines bestimmten Durchmessers der Saugdüse ankäme. Wenn der Konus als Außenkonus ausgebildet ist, wirkt er mit dem Innendurchmesser bzw. der Innenkante der Saugdüse lokalisierend und gegebenenfalls abdichtend zusammen.

In manchen Fällen kann es auch zweckmäßig sein, die Saugdüse während der Verwendung des Instruments bleibend mit diesem zu verbinden. Die Absaugöffnung ist dann mit einer Kupplungseinrichtung zum Ankuppeln der Saugdüse ausgerüstet.

Die Erfindung wird im folgenden näher unter Bezugnahme auf die Zeichnung erläutert, die vorteilhafte Ausführungsbeispiele schematisch veranschaulicht. Es zeigen:
- Fig. 1: eine Seitenansicht einer Gewebestanze und
- Fig. 2 bis 5: unterschiedliche Ausführungsformen der Absaugöffnung und deren Saugdüsenansatzfläche.

Das Instrument umfaßt eine erste Stange 1, die mit einem ersten Handhebel 2 starr verbunden ist, sowie eine zweite Stange 4, die an der ersten Stange 1 durch Führungsmittel 5 in Pfeilrichtung 10 längsverschieblich gelagert und mittels eines beweglichen, zweiten Handhebels 3 vor- und zurückschiebbar ist. Durch die Kraft einer Feder wird die zweite Stange 4 in die dargestellte, zurückgezogene Stellung gedrängt. Durch Zurückziehen des Hebels 3 wird sie vorgeschoben. Sie trägt am vorderen Ende eine U-förmige Schneide 6, deren Ebene parallel ist zu der ihr zugewandten Fläche eines mit ihr zusammenwirkenden Stanzelements 7, das als Gegenplatte ausgebildet ist. An die Schneide 6 schließt sich nach hinten ein Kanal 8 an, der innerhalb der zweiten Stange 4 als Nut ausgebildet ist, deren Öffnungsseite durch die Stange 1 geschlossen wird. Der Kanal 8 endet an seinem hinteren Ende in einer Öffnung 9. Die Stangen können zur Reinigung und Wartung des Instruments voneinander gelöst werden. Insoweit wird auf bekannte Technik zurückgegriffen, die hier nicht erläutert werden muß.

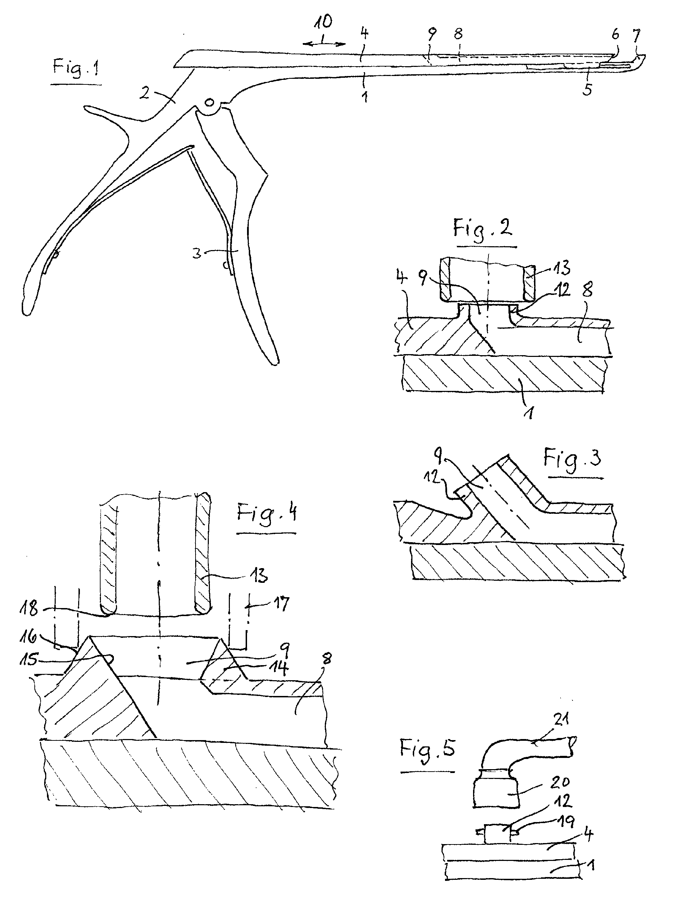

Während der Benutzung sammelt sich im Kanal 8 abgetrenntes Gewebe an, das daraus entfernt werden muß. Dies kann auf verschiedene Weise geschehen. Für den Fall, daß es aus der Öffnung 9 abgesaugt werden soll, schafft die Erfindung durch die in den Fig. 2 bis 5 dargestellten Saugdüsenansatzflächen eine Erleichterung.

Gemäß Fig. 2 wird die Absaugöffnung von einem hohlzylindrischen Rand oder Stutzen 12 gebildet, der über die sonstige Oberfläche der Stange 4 ein wenig hinausragt. Sein Außendurchmesser ist etwas geringer gewählt als der Innendurchmesser der Saugdüse 13 üblicher Absauggeräte. Die Saugdüse 13 kann dadurch bequem auf der die Saugdüsenansatzfläche bildenden Umfangsfläche des Stutzens 12 positioniert werden. Dadurch ist ihr Saugquerschnitt optimal zur Absaugöffnung 9 gelegen. Eine gute Absaugwirkung wird auf diese Weise auch dann erreicht, wenn die Durchmesser des Stutzens 12 und der Saugdüse 13 nicht passend aufeinander abgestimmt sind. Wenn eine solche passende Abstimmung vorhanden ist, ist die Wirkung noch besser.

Nicht gezeigt ist eine ebenfalls mögliche Ausführung, bei welcher der Innendurchmesser des Stutzens 12 größer ist als der Außendurchmesser der Saugdüse 13. Man versteht ohne weiteres, daß auch diese Konfigurationsmöglichkeit den angestrebten Zielen dienen kann, indem die Innenumfangsfläche des Stutzens die Saugdüsenansatzfläche bildet.

Die Ausführung gemäß Fig. 3 gleicht im Prinzip derjenigen gemäß Fig. 2. Der Unterschied besteht darin, daß die Richtung des Stutzens 12 gegenüber der Richtung der Stange 4 geneigt ist. Dies ergibt zum einen einen ungestörteren Materialfluß in der Absaugöffnung 9 als auch eine bequemere Ansatzrichtung für das Absauggerät.

Im Ausführungsbeispiel gemäß Fig. 4 ist die Absaugöffnung 9 von einem zweckmäßigerweise kreisringförmigen Wulst 14 umgeben, der innen eine hohlkonische Fläche und außen eine außen konische Fläche 16 bildet. Mit der hohlkonischen Fläche 15 als Saugdüsenansatzfläche kann der Rand 18 einer Saugdüse 13 sowohl positionierend als auch weitgehend abdichtend zusammenwirken. Entsprechendes gilt für die außenkonische Fläche 16 für einen strichpunktiert angedeuteten Saugstutzen 17, dessen Durchmesser entsprechend größer ist als derjenige der Saugdüse 13.

Im Beispiel gemäß Fig. 5 ist angedeutet, daß der Stutzen 12 mit Bajonettstiften 19 versehen ist, die mit entsprechenden Bajonettnuten eines Kupplungsstücks 20 am Ende eines Absaugschlauchs 21 zusammenwirken können. Dies erlaubt es, den Absaugschlauch 21 während der Operation gewünschtenfalls mit der Absaugöffnung zu verbinden, um eine ständige Absaugung des entfernten Gewebes zu ermöglichen.

Die in den Beispielen gezeigten Saugstutzenansatzflächen umgeben jeweils die Absaugöffnung 9. In manchen Fällen kann eine einseitige Anordnung einer solchen Fläche genügen, um die Positionierung des Saugstutzens hinreichend genau zu ermöglichen.

## Patentansprüche

1. Chirurgische Gewebestanze mit einem eine Absaugöffnung (9) aufweisenden Gewebeaufnahmekanal (8), **dadurch gekennzeichnet, daß** die Absaugöffnung (9) mit einer Saugdüsenansatzfläche (15, 16) versehen ist.

2. Gewebestanze nach Anspruch 1, **dadurch gekennzeichnet, daß** die Saugdüsenansatzfläche von einem vorragenden Stutzen (12) gebildet ist.

3. Gewebestanze nach Anspruch 1 oder 2, **dadurch gekennzeichnet, daß** die Saugdüsenansatzfläche (15, 16) konisch ausgebildet ist.

4. Gewebestanze nach einem der Ansprüche 1 bis 3, **dadurch gekennzeichnet, daß** die Absaugöffnung (9) mit einer Kupplungseinrichtung (19) zum Ankuppeln eines Absauggeräts (21) ausgerüstet ist.

5. Instrumentensatz, bestehend aus einer Gewebestanze mit einem eine Absaugöffnung (9) aufweisenden Gewebeaufnahmekanal (8) und einem Absauggerät mit Saugdüse (13, 17), **dadurch gekennzeichnet, daß** die Saugdüse und eine Saugdüsenansatzfläche (15, 16) der Absaugöffnung (9) passend zueinander ausgebildet sind.
